(19) Europäisches Patentamt
European Patent Office
Office européen des brevets

(11) Veröffentlichungsnummer: **0 288 809 B1**

(12) **EUROPÄISCHE PATENTSCHRIFT**

(45) Veröffentlichungstag der Patentschrift: **05.08.92**

(51) Int. Cl.⁵: **C12N 15/62**, C07K 13/00, C12P 21/02, A61K 37/02

(21) Anmeldenummer: **88105693.1**

(22) Anmeldetag: **09.04.88**

# (54) Bifunktionelle Proteine.

(30) Priorität: **16.04.87 DE 3712985**

(43) Veröffentlichungstag der Anmeldung:
**02.11.88 Patentblatt 88/44**

(45) Bekanntmachung des Hinweises auf die Patenterteilung:
**05.08.92 Patentblatt 92/32**

(84) Benannte Vertragsstaaten:
**AT BE CH DE ES FR GB GR IT LI LU NL SE**

(56) Entgegenhaltungen:
**EP-A- 0 136 489** **EP-A- 0 142 268**
**EP-A- 0 158 198** **EP-A- 0 163 249**
**EP-A- 0 183 350** **EP-A- 0 194 818**
**EP-A- 0 227 938** **EP-A- 0 228 018**
**WO-A-85/04673** **WO-A-87/02060**

(73) Patentinhaber: **HOECHST AKTIENGESELLSCHAFT**
**Postfach 80 03 20**
**W-6230 Frankfurt am Main 80(DE)**

(72) Erfinder: **Habermann, Paul, Dr.**
**Heimchenweg 80**
**W-6230 Frankfurt am Main 80(DE)**

EP 0 288 809 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Interleukin-2, im folgenden IL-2, wirkt als T-Zellwachstumsfaktor. IL-2 verstärkt die Aktivität von "Killer"-Zellen wie NK (Natural Killer)-Zellen, zytotoxischen T-Zellen und LAK (Lymphokine Activated Killer)-Zellen.

Granulozyten-Makrophagen-"Colony Stimulating"-Faktor, im folgenden GM-CSF, hingegen stimuliert die Granulozyten-und Makrophagenbildung aus hämatopoetischen Vorläuferzellen. Die Kombination der beiden biologischen Aktivitäten ist für die Tumorbehandlung mit und ohne Zytostatikagabe interessant. IL-2 und GM-CSF sind jedoch unterschiedlich stabil, was zu Problemen bei der direkten Verabreichung der beiden Komponenten und somit zu einer Verminderung des therapeutischen Erfolges führen kann.

Das Problem der unterschiedlichen Stabilität kann erfindungsgemäß dadurch gelöst werden, daß man diese beiden Proteine zu einem bifunktionellen Protein verknüpft.

Es wurden bereits Fusionsproteine der allgemeinen Formel

Met - X - Y - Z (Ia) oder Met - Z - Y - X (Ib)

zur gentechnischen Herstellung von gegebenenfalls modifiziertem GM-CSF vorgeschlagen, in denen X im wesentlichen die Aminosäurefolge der ersten etwa 100 Aminosäuren von vorzugsweise menschlichem IL-2 bedeutet, Y eine direkte Bindung bedeutet, falls die zum gewünschten Protein benachbarte Aminosäure oder Aminosäurenfolge eine Abspaltung des gewünschten Proteins ermöglicht, oder andernfalls ein Brückenglied aus einer oder mehreren genetisch codierbaren Aminosäuren, das die Abspaltung ermöglicht, und Z eine Sequenz aus genetisch codierbaren Aminosäuren ist, die das gewünschte GM-CSF-Protein darstellt. Man kann hierbei auch die für IL-2 codierende DNA-Sequenz - mehr oder weniger - bis zum Ende nutzen und so - gegebenenfalls modifiziertes - biologisch aktives IL-2 als "Nebenprodukt" erzeugen (nicht vorveröffentlichte Europäische Patentanmeldung mit der Veröffentlichungsnummer (EP-A) 0 228 018 bzw. Südafrikanisches Patent 86/9557).

Im Gegensatz zu dem früheren Vorschlag bezieht sich die Erfindung nicht auf die Verwendung der Proteine als Zwischenprodukt, sondern auf die Anwendung in Verfahren zur therapeutischen Behandlung des menschlichen Körpers bzw. auf Arzneimittel, die solche Fusionsproteine enthalten oder aus solchen Fusionsproteinen bestehen. Ein weiterer Aspekt der Erfindung betrifft die Verwendung dieser Fusionsproteine zur Herstellung eines Arzneimittels zur Behandlung maligner Neubildungen.

Das erfindungsgemäß eingesetzte Fusionsprotein besteht also aus zwei biologisch aktiven Komponenten, und zwar einerseits aus einem IL-2-Anteil, der in an sich bekannter Weise modifiziert sein kann, und andererseits aus einem GM-CSF-Anteil, der ebenfalls modifiziert sein kann, und gegebenenfalls einem Brückenglied entsprechend der Definition Y in den vorstehend angegebenen Formeln. Vorzugsweise entspricht die Anordnung der beiden Komponenten der Formel I a. Das erfindungsgemäße Prinzip kann auch zur Herstellung anderer neuartiger bifunktioneller Proteine dienen.

Die Figur zeigt die Konstruktion des Plasmids pB30, das für ein erfindungsgemäßes bifunktionelles Protein codiert.

Modifikationen des IL-2-Moleküls sind bekannt, wobei hier beispielhaft nur auf die EP-A 0 091 539, 0 109 748, 0 118 617, 0 136 489 und 0 163 249 verwiesen sein soll.

Weiterhin wurde in der nicht vorveröffentlichten EP-A 0 219 839 ein IL-2-Derivat vorgeschlagen, bei dem N-terminal die ersten sieben Aminosäuren deletiert sind.

Modifikationen des GM-CSF-Moleküls wurden in der EP-A 0 228 018 vorgeschlagen.

Weitere Abwandlungen der beiden aktiven Molekülanteile können in an sich bekannter Weise vorgenommen werden, wobei hier beispielhaft nur die spezifische Mutagenese erwähnt sei.

Das Brückenglied Y hat vorteilhaft die Formel II

- Asp - $(As)_x$ - Pro - (II)

in der x eine ganze Zahl bis zu etwa 20 und As eine beliebige, genetisch codierbare Aminosäure mit Ausnahme von Cystein bedeuten.

Vorteilhaft ist in der Formel II am linken Ende der IL-2-Anteil und folglich am rechten Ende der GM-CSF-Anteil angeordnet.

Besonders bevorzugte Ausgestaltungen von Y weisen die Aminosäurenfolge

-Asp-Pro-Met-Ile-Thr-Thr-Tyr-Ala-Asp-Asp-Pro- oder
-Asp-Pro-Met-Ile-Thr-Thr-Tyr-Leu-Glu-Glu-Leu-Thr-Ile-Asp-Asp-Pro-
auf, wobei ebenfalls vorzugsweise der IL-2-Anteil am linken Ende und der GM-CSF-Anteil am rechten Ende angeordnet sind.

Die Expression der erfindungsgemäßen bifunktionellen Proteine kann in an sich bekannter Weise erfolgen. In bakteriellen Expressionssystemen kann der Weg der Direktexpression eingeschlagen werden. Hierfür eignen sich alle bekannten Wirts-Vektor-Systeme mit Wirten wie Bakterien der Arten Streptomyces, B. subtilis, Salmonella typhimurium oder Serratia marcescens, insbesondere E. coli.

Die DNA-Sequenz, die für das gewünschte Protein codiert, wird in bekannter Weise in einen Vektor eingebaut, der in dem gewählten Expressionssystem eine gute Expression gewährleistet.

Hierbei wählt man zweckmäßig den Promotor und Operator aus der Gruppe trp, lac, tac, $P_L$ oder $P_R$ des Phagen λ, hsp, omp oder einen synthetischen Promotor, wie sie beispielsweise in der deutschen Offenlegungsschrift 34 30 683 bzw. in der EP-A 0 173 149 beschrieben sind. Vorteilhaft ist die tac-Promotor-Operator-Sequenz, die inzwischen handelsüblich ist (z. B. Expressionsvektor pKK223-3, Pharmacia, "Molecular Biologicals, Chemicals and Equipment for Molecular Biology", 1984, S. 63).

Bei der Expression des erfindungsgemäßen Proteins kann es sich als zweckmäßig erweisen, einzelne Tripletts der ersten Aminosäuren nach dem ATG-Start-Codon zu verändern, um eine eventuelle Basenpaarung auf der Ebene der mRNA zu verhindern. Solche Veränderungen wie Deletionen oder Additionen einzelner Aminosäuren sind dem Fachmann geläufig und ebenfalls Gegenstand der Erfindung.

Für die Expression in Hefen - bevorzugt S. cerevisiae -verwendet man zweckmäßig ein Sekretionssystem, beispielsweise die heterologe Expression über das α-Faktor-System, das mehrfach beschrieben ist.

Für die Expression des bifunktionellen Moleküls in Hefe ist es vorteilhaft, wenn in dem bifunktionellen Protein dibasische Peptidsequenzen sowie Glykosylierungsstellen durch entsprechenden Austausch einzelnen Aminosäuren zerstört werden. Hierbei ergeben sich viele Kombinationsmöglichkeiten, die auch die biologische Wirkung beeinflussen können.

Die Expression von IL-2 in Hefe ist aus der EP-A 0 142 268 bekannt, von GM-CSF aus der EP-A 0 188 350.

Die Verabreichung der erfindungsgemäßen bifunktionellen Proteine entspricht derjenigen der beiden Komponenten. Wegen der größeren Stabilität ist jedoch in vielen Fällen eine geringere Dosierung möglich, die sich im unteren Bereich der bisher vorgeschlagenen Dosierungen hält.

In den folgenden Beispielen wird die Erfindung näher erläutert. Prozentangaben und Relationen beziehen sich auf das Gewicht, sofern keine anderen Angaben gemacht sind.

Beispiel 1

Das Plasmid p159/6 (EP-A2 0 163 249, Figur 5; (1) in der vorliegenden Figur) enthält zwischen einer EcoRI- und einer SalI-Schnittstelle ein synthetisches, für IL-2 codierendes Gen. Die DNA-Sequenz für dieses Gen ist in der genannten EP-A2 als "DNA-Sequenz I" wiedergegeben. Im Bereich der Tripletts 127 und 128 befindet sich eine TaqI-Schnittstelle. Aus diesem Plasmid wird durch Schneiden mit EcoRI und TaqI die IL-2-Teilsequenz (2) herausgeschnitten und isoliert.

Aus der EP-A2 0 183 350 ist das Plasmid pHG23 (3) bekannt, das für GM-CSF codiert. Die GM-CSF-cDNA ist in Figur 2 dieser EP-A2 wiedergegeben. Das Plasmid pHG23 wird erhalten, wenn man die cDNA-Sequenz in die PstI-Schnittstelle von pBR322 einbaut, wobei einerseits von der PstI-Schnittstelle beim 5′-Ende und andereseits von einer durch GC-"Tailing" eingeführten PstI-Stelle am 3′-Ende Gebrauch gemacht wird. Aus diesem Plasmid wird durch Schneiden mit SfaNI und PstI die DNA-sequenz (4) isoliert, die den größen Teil des GM-CSF-Gens enthält.

Nach der Phosphit-Methode wird das folgende Oligonukleotid (5) synthetisiert:

```
      128                       (133)
      Ile  Ile  Ser  Thr  Leu  Asp  Pro  Met  Ile
   CG ATC  ATC  TCT  ACC  CTG  GAC  CCG  ATG  ATC
      TAG  TAG  AGA  TGG  GAC  CTG  GGC  TAC  TAG
  (TaqI)                                                  (5)
                                          1     2
      Thr  Thr  Tyr  Ala  Asp  Asp  Pro  (Ala) (Pro)
      ACC  ACC  TAT  GCG  GAC  GAT  CCG  GC
      TGG  TGG  ATA  CGC  CTG  CTA  GGC  CGT   GGG
                                         (SfaNI)
```

Das Oligonukleotid (5) ergänzt am 5'-Ende die DNA-Sequenz von IL-2, wobei jedoch in Position 133 anstelle von Thr Asp steht. Am 3'-Ende dieses Oligonukleotids finden sich die Nukleotide, die durch Schneiden mit SfaNI aus der cDNA weggefallen sind.

Die Herstellung des Expressionsplasmids pEW1000 (6) ist in der (nicht vorveröffentlichten) EP-A 0 227 938 vorgeschlagen (Fig. 1). Dieses Plasmid ist ein Derivat des Plasmids ptac 11 (Amann et al., Gene 25 (1983) 167 - 178), bei dem in die Erkennungsstelle für EcoRI eine synthetische Sequenz eingebaut wurde, die eine SalI-Schnittstelle enthält. Man erhält so das Expressionsplasmid pKK 177.3. Durch Insertion des lac-Repressors (Farabaugh, Nature 274 (1978) 765 - 769) erhält man das Plasmid pJF118. Dieses wird an der singulären Restriktionnschnittstelle für AvaI geöffnet und in bekannter Weise durch Exonuklease-Behandlung um etwa 1000 bp verkürzt und ligiert. Man erhält das Plasmid pEW1000 (6). Durch Öffnen dieses Plasmids im Polylinker mit den Enzymen EcoRI und PstI erhält man das linearisierte Expressionsplasmid (7).

Diese linearisierte Plasmid-DNA (7) wird nun mit dem DNA-Fragment (2), das für die IL-2-Sequenz codiert, mit dem synthetischen Oligonukleotid (5) und mit dem cDNA-Fragment (4) ligiert. Es entsteht das Plasmid pB30 (8), das in den E. coli-Stamm Mc1061 transformiert wird. Die Plasmid-DNA einzelner Klone wird isoliert und durch Restriktionsanalyse charakterisiert.

Beispiel 2

Setzt man im Beispiel 1 anstelle des Oligonukleotids (5) das folgende synthetische Oligonukleotid

```
      128                       (133)
      Ile  Ile  Ser  Thr  Leu  Asp  Pro  Met  Ile  Thr  Thr  Tyr
   CG ATC  ATC  TCT  ACC  CTG  GAC  CCG  ATG  ATC  ACC  ACC  TAT
      TAG  TAG  AGA  TGG  GAC  CTG  GGC  TAC  TAG  TGG  TGG  ATA
  (TaqI)
                                                    1     2
      Leu  Glu  Glu  Leu  Thr  Ile  Asp  Asp  Pro  (Ala) (Pro)
      CTA  GAA  GAG  CTC  ACG  ATC  GAC  GAT  CCG  GC
      GAT  CTT  CTC  GAG  TGC  TAG  CTG  CTA  GGC  CGT   GGG
                                                   (SfaNI)
```

ein, so erhält man das Plasmid pB31.

Beispiel 3

Kompetente Zellen des E. coli-Stammes W3110 werden mit dem Plasmid pB30 oder pB31 transformiert. Eine Übernachtkultur des Stammes wird mit LB-Medium (J.H. Miller, Experiments in Molec. Gen., Cold Spring Harbor Lab., 1972), das 50 µg/ml Ampicillin enthält, im Verhältnis von etwa 1:100 verdünnt und das Wachstum über OD-Messung verfolgt. Bei OD = 0,5 wird die Kultur auf eine Konzentration von 2mM Isopropyl-β-D-thiogalactopyranosid (IPTG) eingestellt und die Bakterien werden nach 150 - 180 Minuten abzentrifugiert. Die Bakterien werden ca. 5 Minuten in einer Puffermischung (7M Harnstoff, 0,1 % SDS, 0, 1M Natriumphosphat, pH 7,0) behandelt und Proben auf eine SDS-Polyacrylamid-Gelelektrophoreseplatte aufgetragen. Die Expression des bifunktionellen Proteins wird so bestätigt.

Die angegebenen Bedingungen gelten für Schüttelkulturen; bei größeren Fermentationen sind entsprechend veränderte OD-Werte, Nährmedien und variierte IPTG-Konzentrationen zweckmäßig.

Beispiel 4

Nach Induktion werden E. coli W3110-Zellen, die das Plasmid pB30 oder pB31 enthalten, abzentrifugiert, in Natriumphosphatpuffer (pH 7) resuspendiert und erneut abzentrifugiert. Die Bakterien werden in dem gleichen Puffer aufgenommen und anschließend aufgeschlossen (French Press, ®Dyno-Mühle). Der Zellaufschluß wird abzentrifugiert. Überstand und Sediment werden mittels SDS-Polyacrylamidgelelektrophorese wie im Beispiel 3 beschrieben analysiert. Nach Anfärben der Proteinbanden zeigt sich, daß das bifunktionelle Protein im Sediment des Aufschlusses gefunden wird. Das Sediment wird mehrmals mit chaotropen Puffern und zuletzt mit Wasser gewaschen, wobei das gewünschte Protein weiter angereichert wird. In der wäßrigen Proteinsuspension wird anschließend die Proteinkonzentration bestimmt. Die Suspension wird nun auf eine Konzentration von 5M Guanidiniumhydrochlorid und 2 mM Dithiothreitol (DTT) eingestellt. Das Gemisch wird ca. 30 Minuten unter Stickstoff gerührt und anschließend mit 50 mM Tris-Puffer (pH 8,5) so verdünnt, daß die Proteinkonzentration 100 µg/ml ergibt. Nun wird gegen diesen Tris-Puffer dialysiert und nach 2-maligem Pufferwechsel gegen Wasser dialysiert. Das so behandelte Protein wird steril filtriert und auf seine biologische Aktivität überprüft. Es zeigt sowohl im Interleukin-2-abhängigen CTLL 2-Zellproliferationstest als auch im humanen Knochenmarkstest volle biologische Wirkung. Gemischte Kolonien aus Granulozyten und Makrophagen werden dabei beobachtet.

Das bifunktionelle Protein kann über Interleukin-2-spezifische Affinitätschromatographie weiter gereinigt werden. Das Protein ist auch dann in beiden Tests aktiv. Ein E. coli-Extrakt des nichttransformierten Stammes W3110, der wie beschrieben behandelt wurde, zeigt dagegen keine Aktivität.

Für die technische Herstellung des Produktes sind andere Bedingungen zweckmäßig, beispielsweise für die Faltung des Proteins und seine Reinigung. Als - an sich bekannte -Reinigungsverfahren kommen Ionentausch, Adsorption, Gelfiltration und präparative HPLC-Chromatographie in Betracht.

## Patentansprüche

## Patentansprüche für folgende Vertragsstaaten : AT, BE, CH, DE, FR, GB, IT, LI, LU, NL, SE

**1.** Bifunktionelle Proteine, bestehend aus einem biologisch aktiven Interleukin-2 (IL-2)- und Granulozyten-Makrophagen-"Colony Stimulating"-Faktor (GM-CSF)-Anteil, zur Anwendung als Arzneimittel.

**2.** Bifunktionelle Proteine mit einem biologisch aktiven IL-2- und GM-CSF-Anteil, dadurch gekennzeichnet, daß die beiden biologisch aktiven Protein-Anteile über eine Brücke aus 1 bis etwa 20 genetisch codierbaren Aminosäuren verknüpft sind.

**3.** Protein nach Anspruch 2, dadurch gekennzeichnet, daß die Brücke der Formel (II)

$$- Asp - (As)_x - Pro - \qquad (II)$$

entspricht, wobei x eine ganze Zahl von 1 bis 18 bedeutet und As eine genetisch codierbare Aminosäure mit Ausnahme von Cys ist.

**4.** Protein nach Anspruch 3, dadurch gekennzeichnet, daß das Brückenglied $(As)_x$ die Aminosäurenfolge

-Pro-Met-Ile-Thr-Thr-Tyr-Ala-Asp-Asp-

oder

-Pro-Met-Ile-Thr-Thr-Tyr-Leu-Glu-Glu-Leu-Thr-Ile-Asp-Asp-

bedeutet.

5. Protein nach einem oder mehreren der Ansprüche 2 bis 4, dadurch gekennzeichnet, daß N-terminal der IL-2-Anteil und C-terminal der GM-CSF-Anteil angeordnet ist.

6. Verfahren zur Herstellung von bifunktionellen Proteinen nach Anspruch 1 bis 5, dadurch gekennzeichnet, daß man ein für diese Proteine codierendes Gen konstruiert und in einer Wirtszelle exprimiert.

7. Arzneimittel, bestehend aus einem Protein nach Anspruch 1 bis 5, gegebenenfalls in Kombination mit einem pharmakologisch geeigneten Träger

8. Verwendung eines Proteins nach Anspruch 1 bis 5 zur Herstellung eines Arzneimittels zur Behandlung maligner Neubildungen.

**Patentansprüche für folgende Vertragsstaaten : ES, GR**

1. Verfahren zur Herstellung von bifuntionellen Proteinen bestehend aus einem biologisch aktiven Interleukin-2 (IL-2)- und Granulozyten-Makrophagen-"Colony Stimulating"-Faktor (GM-CSF)-Anteil, wobei die beiden biologisch aktiven Protein-Anteile über eine Brücke aus 1 bis etwa 20 genetisch codierbaren Aminosäuren verknüpft sind, dadurch gekennzeichnet, daß man ein für diese Proteine codierendes Gen konstruiert und in einer Wirtszelle exprimiert.

2. Verfahren nach Anspruch 1, dadurch gekennzeichnet, daß die Brücke der Formel

   - Asp - $(As)_x$ - Pro -

   entspricht, wobei x eine ganze Zahl von 1 bis 18 bedeutet und As eine genetisch codierbare Aminosäure mit Ausnahme von Cys ist.

3. Verfahren nach Anspruch 2, dadurch gekennzeichnet, daß das Brückenglied $(As)_x$ die Aminosäurenfolge

-Pro-Met-Ile-Thr-Thr-Tyr-Ala-Asp-Asp-

oder

-Pro-Met-Ile-Thr-Thr-Tyr-Leu-Glu-Glu-Leu-Thr-Ile-Asp-Asp-

bedeutet.

4. Verfahren nach einem oder mehreren der vorhergehenden Ansprüche 2 bis 4, dadurch gekennzeichnet, daß N-terminal der IL-2-Anteil und C-terminal der GM-CSF-Anteil angeordnet ist.

**Claims**

**Claims for the following Contracting States : AT, BE, CH, DE, FR, GB, IT, LI, LU, NL, SE**

1. A bifunctional protein composed of a biologically active interleukin-2 (IL-2) constituent and granulocyte macrophage colony stimulating factor (GM-CSF) constituent, for use as medicament.

2. A bifunctional protein having a biologically active IL-2 constituent and GM-CSF constituent, wherein the two biologically active protein constituents are linked via a bridge composed of 1 to about 20 genetically encodable amino acids.

3. A protein as claimed in claim 2, wherein the bridge corresponds to the formula (II)

- Asp - $(aa)_x$ - Pro - (II)

x denoting an integer from 1 to 18, and aa being a genetically encodable amino acid with the exception of Cys.

4. A protein as claimed in claim 3, wherein the bridging member $(aa)_x$ denotes the amino acid sequence

-Pro-Met-Ile-Thr-Thr-Tyr-Ala-Asp-Asp-

or

-Pro-Met-Ile-Thr-Thr-Tyr-Leu-Glu-Glu-Leu-Thr-Ile-Asp-Asp-.

5. A protein as claimed in one or more of claims 2 to 4, wherein the IL-2 constituent is arranged N-terminal and the GM-CSF constituent is arranged C-terminal.

6. A process for the preparation of bifunctional proteins as claimed in any of claims 1 to 5, which comprises construction, and expression in a host cell, of a gene coding for these proteins.

7. A medicament composed of a protein as claimed in any of claims 1 to 5, where appropriate combined with a pharmacologically suitable vehicle.

8. The use of a protein as claimed in any of claims 1 to 5 for the preparation of a medicament for the treatment of malignant neoplasms.

**Claims for the following Contracting States : ES, GR**

1. A process for the preparation of bifunctional proteins composed of a biologically active interleukin-2 (IL-2) constituent and granulocyte macrophage colony stimulating factor (GM-CSF) constituent, where the two biologically active protein constituents are linked via a bridge composed of 1 to about 20 genetically encodable amino acids which comprises construction, and expression in a host cell, of a gene coding for these proteins.

2. The process as claimed in claim 1, wherein the bridge corresponds to the formula

- Asp - $(aa)_x$ - Pro -

x denoting an integer from 1 to 18, and aa being a genetically encodable amino acid with the exception of Cys.

3. The process as claimed in claim 2, wherein the bridging member $(aa)_x$ denotes the amino acid sequence

-Pro-Met-Ile-Thr-Thr-Tyr-Ala-Asp-Asp-

or

-Pro-Met-Ile-Thr-Thr-Tyr-Leu-Glu-Glu-Leu-Thr-Ile-Asp-Asp-.

**4.** The process as claimed in one or more of the preceding claims 2 to 4, wherein the IL-2 constituent is arranged N-terminal and the GM-CSF constituent is arranged C-terminal.

**Revendications**

**Revendications pour les Etats contractants suivants : AT, BE, CH, DE, FR, GB, IT, LI, LU, NL, SE**

**1.** Protéines bifonctionnelles, constituées d'une fraction biologiquement active d'interleukine-2 (IL-2) et de facteur de stimulation de colonies de macrophages granulocytes (GM-CSF), pour l'utilisation en tant que médicament.

**2.** Protéines bifonctionnelles ayant une fraction biologiquement active d'IL-2 et de GM-CSF, caractérisées en ce que les deux fractions protéiques biologiquement actives sont reliées par un pont composé de 1 à 20 aminoacides codables par voie génétique.

**3.** Protéine selon la revendication 2, caractérisée en ce que le pont correspond à la formule (II)

- Asp - $(As)_x$ - Pro - (II),

x représentant un nombre entier allant de 1 à 18 et As étant un aminoacide codable par voie génétique, à l'exception de Cys.

**4.** Protéine selon la revendication 3, caractérisée en ce que l'élément de pont $(As)_x$ représente la séquence d'aminoacides
-Pro-Met-Ile-Thr-Thr-Tyr-Ala-Asp-Asp-
ou
-Pro-Met-Ile-Thr-Thr-Tyr-Leu-Glu-Glu-Leu-Thr-Ile-Asp-Asp-.

**5.** Protéine selon une ou plusieurs des revendications 2 à 4, caractérisée en ce que la fraction d'IL-2 est placée en position N-terminale et la fraction de GM-CSF est placée en position C-terminale.

**6.** Procédé pour préparer des protéines bifonctionnelles selon les revendications 1 à 5, caractérisé en ce que l'on construit un gène codant pour ces protéines et on l'exprime dans une cellule-hôte.

**7.** Médicament, constitué d'une protéine selon les revendications 1 à 5, éventuellement combiné à un véhicule pharmacologiquement approprié.

**8.** Utilisation d'une protéine selon les revendications 1 à 5 pour la fabrication d'un médicament destiné au traitement de néoformations malignes.

**Revendications pour les Etats contractants suivants : ES, GR**

**1.** Procédé pour préparer des protéines bifonctionnelles, constituées d'une fraction biologiquement active d'interleukine-2 (IL-2) et de facteur de stimulation de colonies de macrophages granulocytes (GM-CSF), les deux fractions protéiques biologiquement actives étant reliées par un pont composé de 1 à 20 aminoacides codables par voie génétique, caractérisé en ce que l'on construit un gène codant pour ces protéines et on l'exprime dans une cellule-hôte.

**2.** Procédé selon la revendication 1, caractérisé en ce que le pont correspond à la formule (II)

- Asp - $(As)_x$ - Pro - (II),

x représentant un nombre entier allant de 1 à 18 et As étant un aminoacide codable par voie génétique, à l'exception de Cys.

**3.** Procédé selon la revendication 2, caractérisé en ce que l'élément de pont $(As)_x$ représente la séquence d'aminoacides
-Pro-Met-Ile-Thr-Thr-Tyr-Ala-Asp-Asp-
ou
-Pro-Met-Ile-Thr-Thr-Tyr-Leu-Glu-Glu-Leu-Thr-Ile-Asp-Asp-.

**4.** Procédé selon une ou plusieurs des revendications 1 à 3, caractérisé en ce que la fraction d'IL-2 est placée en position N-terminale et la fraction de GM-CSF est placée en position C-terminale.

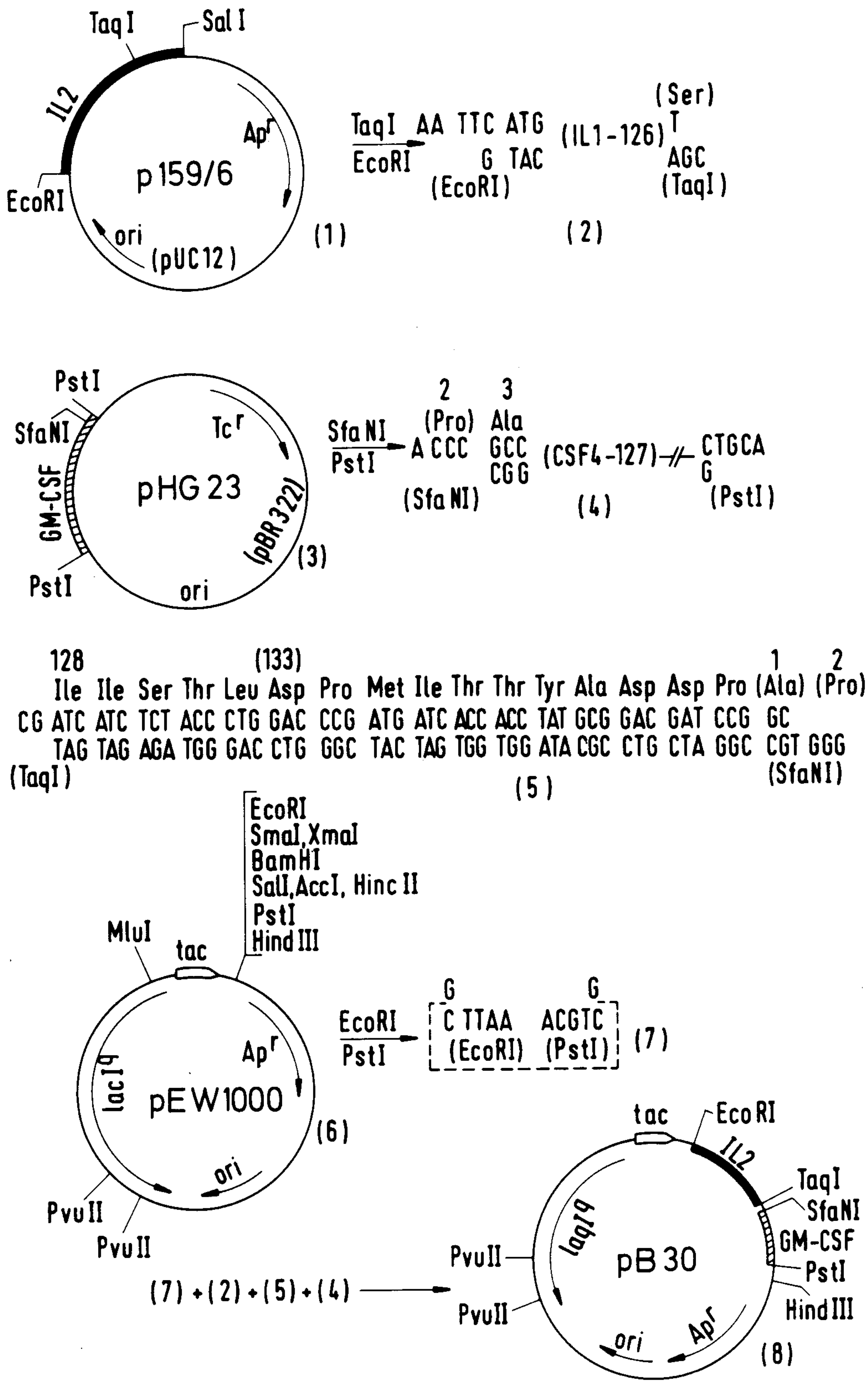
TaqI
SalI
IL2
Ap^r
p159/6
EcoRI
ori
(pUC12)
(1)
TaqI
EcoRI
AA TTC ATG
G TAC
(EcoRI)
(IL1-126)
(Ser)
T
AGC
(TaqI)
(2)
PstI
SfaNI
GM-CSF
Tc^r
pHG 23
(pBR322)
PstI
ori
(3)
SfaNI
PstI
2
(Pro)
A CCC
(SfaNI)
3
Ala
GCC
CGG
(CSF4-127)
CTGCA
G
(PstI)
(4)
128 (133) 1 2
Ile Ile Ser Thr Leu Asp Pro Met Ile Thr Thr Tyr Ala Asp Asp Pro (Ala) (Pro)
CG ATC ATC TCT ACC CTG GAC CCG ATG ATC ACC ACC TAT GCG GAC GAT CCG GC
TAG TAG AGA TGG GAC CTG GGC TAC TAG TGG TGG ATA CGC CTG CTA GGC CGT GGG
(TaqI)
(5)
(SfaNI)
EcoRI
SmaI, XmaI
BamHI
SalI, AccI, Hinc II
PstI
Hind III
MluI
tac
Ap^r
lacI^q
pEW1000
ori
PvuII
PvuII
(6)
EcoRI
PstI
G
C TTAA
(EcoRI)
G
ACGTC
(PstI)
(7)
tac
EcoRI
IL2
TaqI
SfaNI
GM-CSF
PstI
HindIII
lacI^q
pB 30
PvuII
PvuII
ori
Ap^r
(8)
(7) + (2) + (5) + (4)